# EUROPEAN PATENT APPLICATION

(11) **EP 4 082 487 A1**
(43) Date of publication of application: **02.11.2022**
(21) Application number: 22170680.7
(22) Date of filing: 29.04.2022
(51) Int. Cl.: A61F 2/40, A61F 2/30, A61B 17/00

(54) **SPACER IMPLANT SYSTEMS**

(30) Priority: 30.04.2021 US 202117245327
(71) Applicant: Medos International Sarl, 2400 Le Locle (CH)
(72) Inventor: SPENCINER, David B., Raynham, 02767 (US); GABRIEL, Stefan M., Raynham, 02767 (US); SENGUN, Mehmet Ziya, Raynham, 02767 (US); BARRY, Donald E., Raynham, 02767 (US); TOKISH, John, Scotssdale, 85255 (US); CHAMBERLAIN, Aaron, St. Louis, 63110 (US); VON RECHENBERG, Annemarie Brigitte, 8903 Birmensdorf (CH); MORODER, Philipp, 14193 Berlin (DE); JACOFSKY, Marc, Phoenix, 85080 (US); MCALISTER, Gary, Raynham, 02767 (US)
(74) Representative: Carpmaels & Ransford LLP

(57) **Abstract**

Implants are provided. In one exemplary embodiment, an implant includes a spacer configured to be implanted within a joint. The spacer includes an elongated central body extending from a first end to a second end with a longitudinal axis extending therebetween, and first and second wings extending from the first and second ends of the elongated central body, respectively. The elongated central body has a first maximum height in a direction transverse to the longitudinal axis, and each of the first and second wings has a maximum height greater than the first maximum height. When implanted and in the inflated state, at least one of the first wing and the second wing is configured to mechanically interlock with a portion of the anatomy to thereby self-anchor the spacer to the joint and inhibit migration of the spacer. Implant systems and methods for biomechanically augmenting muscle function are also provided.

## Description

### FIELD

The present disclosure generally relates to spacer implants and spacer implant systems.

### BACKGROUND

The shoulder joint has the largest range of motion of any joint in the human body. It is a ball-and-socket joint having three bones: a shoulder blade (scapula), a collarbone (clavicle), and an upper arm bone (humerus). A rounded head of the upper arm bone (humeral head) fits into a shallow socket in the shoulder blade called a glenoid. The humeral head is usually much larger than the glenoid, and together they have little inherent stability. The primary stabilizers of the shoulder joint include the biceps brachii on the anterior side of the arm and the tendons of the rotator cuff. The rotator cuff includes four muscles and their tendons: the supraspinatus muscle, the infraspinatus muscle, the teres minor muscle, and the subscapularis muscle.

Under circumstances where the rotator cuff becomes torn (rotator cuff tear), its ability to stabilize the shoulder joint can be compromised. For example, a rotator cuff tear can allow the ball-and-socket joint of the shoulder joint to move upwards from the socket and therefore result in superior migration of the humerus. This can lead to reduced arm function and require treatment.

In the case of a partial tear, the injury will frequently heal itself, if given sufficient time and if care is taken not to expose the injury to further undue stress. In the case of a complete tear, however, surgery is often needed to reattach the rotator cuff to its associated bone or bones, *e.g.,* the humerus and/or the scapula. Numerous devices are currently available to re-attach the rotator cuff to bone. Examples of such currently-available devices include screws, staples, suture anchors, and tacks. While such currently-available devices can be effective in reattaching the rotator cuff to bone, in some cases the rotator cuff tear does not heal completely or the rotator cuff tear recurs after surgery as the patient moves the shoulder in the course of their daily life. The patient therefore may again experience pain and reduced arm function and may require another surgery and/or other treatment to address the deficient rotator cuff. Moreover, it is not uncommon for patients to experience pain at night and, as a result, have difficulty sleeping.

Further, in certain instances, muscle insufficiency can also cause instability of the shoulder joint. This can be caused by a nerve dysfunction or by alterations in natural anatomy such as superior migration of the humerus causing deltoid relaxation. For example, excessive superior migration of the humerus may cause too much laxity in the deltoid and therefore prevent contraction to raise the arm. Thus, a weak deltoid can lead to serious shoulder dysfunction including pseudoparalysis. While implants have been developed to improve muscle insufficiency, such implants are typically rigid and need to be permanently fixed to bone.

Accordingly, there remains a need for improved shoulder repair devices and techniques.

### SUMMARY

Implants are provided. In one example, an implant includes a spacer configured to be implanted within a joint, in which the spacer is configurable between a deflated state and an inflated state. The spacer includes an elongated central body extending from a first end to a second end with a longitudinal axis extending therebetween, and first and second wings extending from the first and second ends of the elongated central body, respectively. The elongated central body has a first maximum height in a direction transverse to the longitudinal axis, and each of the first and second wings has a maximum height greater than the first maximum height. When implanted and in the inflated state, at least one of the first wing and the second wing is configured to mechanically interlock with a portion of the anatomy to thereby self-anchor the spacer to the joint and inhibit migration of the spacer.

The joint can have a variety of configurations. The joint can be a shoulder joint and the spacer can be configured to be implanted between an acromion and a humerus of the shoulder joint and, when in the inflated state, the spacer is configured to at least partially encircle the acromion. A portion of at least one of the first wing and the second wing can be curved upward and away from the longitudinal axis of the elongated central body such that the portion of the at least one of the first wing and the second wing can be configured to extend above a deep-most surface of the acromion when the spacer is implanted and in the inflated state. A portion of at least one of the first wing and the second wing can be curved upward and away from the longitudinal axis of the elongated central body such that the portion of the at least one of the first wing and the second wing can be configured to extend flush to or above a superficial-most surface of the acromion when the spacer is implanted and in an inflated state.

The spacer can have a variety of configurations. The spacer can be substantially U-shaped. Alternatively, the spacer can be substantially dog-bone shaped. At least one surface of the spacer can be textured to thereby further inhibit migration of the spacer when the spacer is implanted.

The spacer can include at least one medicament disposed therein, and the spacer can be configured to release the at least one medicament while the spacer is implanted. The spacer can be configured to receive at least one medicament when being inflated, and the spacer can be configured to release the at least one medicament while the spacer is implanted.

Implant systems are also provided. An implant system includes a first spacer configured to be positioned within a first space of a joint, a second spacer being configured to be positioned within a second space of the joint, a first fluid passageway, and a first valve being positioned within the first fluid passageway. The first spacer has a first inflatable chamber defined therein that is configured to reversibly move between a first volume and a second volume, and the second spacer has a second inflatable chamber defined therein that is configured to reversibly move between a third volume and a fourth volume. The first fluid passageway extends between the first and second inflatable chambers. The first valve being configured to move between at least a first state and a second state that is different than the first state. The first state inhibits fluid transfer between the first and second inflatable chambers to prevent movement between respective volumes, and the second state allows a first fluid transfer to occur between the first and second inflatable chambers based on a first position of the joint such that the first and second inflatable chambers can reversibly move between respective volumes.

The implant system can include at least one pump that can be in fluid communication with the fluid passageway. The at least one pump can be configured to drive fluid flow between the first and second inflatable chambers when the at least one valve is in the second state.

The first valve can have a variety of configurations. The first valve can be configured to facilitate a second, opposite fluid transfer between the first and second inflatable chambers based on a second position of the shoulder joint such that the first and second inflatable chambers can reversibly move between respective volumes. The second position can be different than the first position.

The joint can have a variety of configurations. The joint can be a shoulder joint and the first space can be posterior to a humeral head of the shoulder joint and the second space can be superior or anterior to the humeral head.

The implant system can include a third spacer that can be configured to be positioned within a third space of the joint, a second fluid passageway, and a second valve that can be positioned within the second fluid passageway. The third spacer can have a third inflatable chamber defined therein that can be configured to reversibly move between a fifth volume and a sixth volume. The second fluid passageway can extend between the second and third inflatable chambers. The second valve can be configured to move between at least a third state and a fourth state that is different than the third state. The third state can inhibit fluid transfer between the second and third inflatable chambers to prevent movement between respective volumes, and the fourth state can allow fluid transfer to occur between the second and third inflatable chambers based on at least one of the first position or a third position of the joint such that the second and third inflatable chambers can reversibly move between respective volumes.

The joint can have a variety of configurations. The joint can be the shoulder joint and the first space can be posterior to a humeral head of the shoulder joint, the second space can be superior to the humeral head, and the third space can be anterior to the humeral head.

The implant system can include a main fluid passageway having a central lumen and three branched lumens. The first branched lumen can extend between the central lumen and the first inflatable chamber, the second branched lumen can extend between the central lumen and the second inflatable chamber, and the third branched lumen can extend between the central lumen and the third inflatable chamber. The implant system can include at least one third valve that can be positioned within at least one of the first branched lumen, the second branched lumen, and the third branched lumen, and the at least one third valve can be configured to move between a fifth state that inhibits fluid transfer therethrough and a sixth state that allows fluid therethrough. In such examples, the implant system can include a fourth valve that can be positioned at an end of the central lumen and can be configured to move between a seventh state and an eighth state, and, when in the eighth state, the fourth valve can be configured to allow fluid transfer therethrough to at least one of inflate and deflate at least one of the first, second, and third inflatable chambers when the at least one third valve is in a sixth state.

Methods for biomechanically augmenting muscle function are also provided. The method includes implanting a spacer in a deflated state between a humerus and a muscle; and inflating the spacer from the deflated state to an inflated state so as to allow the spacer to self-anchor between the humerus and the muscle without penetration into the humerus and to lengthen the muscle and increase the compression around a humeral head of the humerus.

The spacer can be positioned lateral to the humerus.

### BRIEF DESCRIPTION OF THE DRAWINGS

This invention will be more fully understood from the following detailed description taken in conjunction with the accompanying drawings, in which:
FIG. 1 is a side view of an implant, showing the implant in an inflated state;
FIG. 2 is a top view of the implant of FIG. 1;
FIG. 3 is a side view of another implant, showing the implant in an inflated state;
FIG. 4 is a side view of a shoulder showing the implant of FIG. 3 implanted in the subacromial space of the shoulder;
FIG. 5 is a top view of the shoulder showing the implant of FIG. 3 implanted in the subacromial space of the shoulder;
FIG. 6 is a side view of another embodiment of an implant, showing the implant in an inflated state and implanted in the subacromial space of a shoulder;
FIG. 7 is a side view of another implant, showing the implant in an inflated state;
FIG. 8 is a top view of the implant of FIG. 7;
FIG. 9 is a top view of a shoulder with another implant, showing the implant in an inflated state and implanted in the subacromial space of a shoulder;
FIG. 10 is a front view of a shoulder with a weak/dysfunctioning deltoid muscle;
FIG. 11 is a front view of the shoulder of FIG. 10 with another an implant positioned between the deltoid and a humerus of the shoulder, showing the implant in an inflated state;
FIG. 12 is a front view of an implant system having first and second spacers;
FIG. 13 is side view of a left scapula of a shoulder joint with the first and second spacers of the implant system of FIG. 12 positioned within respective first and second spaces of the left scapula, showing the first spacer in a deflated state and the second spacer in an inflated state;
FIG. 14 is side view of a left scapula of a shoulder joint with the first and second spacers of the implant system of FIG. 12 positioned within respective first and second spaces of the left scapula, showing the first spacer in an inflated state and the second spacer in a deflated state;
FIG. 15A is another an implant system having three spacers, showing each spacer in a deflated state;
FIG. 15B is the implant of FIG. 15A, showing each spacer in an inflated state;
FIG. 16A is a top view of an anatomical environment illustrating a left humerus and scapula with ribs and part of a spine;
FIG. 16B is a magnified view of a portion of FIG. 16A, further showing the implant system of FIG. 15B implanted between the scapula and the humerus;
FIG. 17A is a left side view of the anatomical environment of FIG. 16A;
FIG. 17B is a magnified view of a portion of FIG. 17A, further showing the implant system of FIG. 15B implanted between the scapula and the humerus;
FIG. 18A is a rear side view of the anatomical environment of FIG. 16A; and
FIG. 18B is a magnified view of a portion of FIG. 18A, further showing the implant system of FIG. 15B implanted between the scapula and the humerus.

### DETAILED DESCRIPTION

Certain examples will now be described to provide an overall understanding of the principles of the structure, function, manufacture, and use of the implants, systems, and methods disclosed herein. One or more examples are illustrated in the accompanying drawings. Those skilled in the art will understand that the implants, systems, and methods specifically described herein and illustrated in the accompanying drawings are non-limiting and that the scope of the present invention is defined solely by the claims. The features illustrated or described in connection with one example may be combined with the features of other examples. Such modifications and variations are intended to be included within the scope of the present invention.

In general, implants, implant systems, and methods of using the same are provided. In one example, an implant is a spacer (*e.g.*, having one or more inflatable chambers) that is configured to be implanted within a joint *(e.g.,* a shoulder joint) to stabilize the joint and it has certain features that are configured to self-anchor thereby inhibiting migration of the spacer. It will be appreciated that absent such self-anchoring, additional anchoring features can be needed to maintain the position of the spacer relative to the joint when implanted. Beneficially, without requiring additional anchoring mechanisms, such as sutures, suture anchors, etc., implantation of the present spacers is less invasive.

In another example, the implant is a spacer (*e.g.,* having one or more inflatable chambers) that is configured to be implanted between bone and muscle (*e.g.,* interbody implantation) or between muscles *(e.g.,* intermuscular implantation) or muscle fibers *(e.g.,* intramuscular implantation) to augment the muscle(s) or muscle fibers so as to improve muscle function. That is, the spacer can be configured for biomechanical augmentation of muscle function. Unlike conventional implants that are typically rigid and require to be permanently affixed to bone (such as by penetrating the bone), the present spacers are designed to be self-anchoring and flexible. As a result, muscle function augmentation with the use of the present spacers can be carried out with less tissue invasion, with flexibility for intra-muscle positioning, and with less or no bone involvement.

The spacer generally includes one or more internal inflatable chambers defined therein and can be configurable between a deflated state and an inflated state. In use, a spacer can be inserted within a joint in its deflated state, positioned within the joint to a desired location *(e.g.,* within the subacromial space), and then inflated *in situ.* The subacromial space refers to the space above the shoulder's glenohumeral joint (ball-and-socket joint) and below the acromion, the superficial-most bony prominence of the scapula of the shoulder. The spacer can be configured to be implanted in a variety of different joints. For example, the joint can be a shoulder joint and the spacer can be configured to be implanted between an acromion and a humerus of the shoulder joint. Further, when in the inflated state, the spacer can be configured to at least partially encircle the acromion.

The spacer can be configured to be inserted using an open procedure, a mini-open procedure, or an all-arthroscopic procedure. For example, the spacer can be inserted into the desired location using a small diameter delivery tube or cannula. Other suitable known methods can be used. The spacer can be configured to be inflated from its deflated state by injecting one or more filling materials into one or more inflatable chambers of the spacer. Any suitable filling method can be used to inflate the spacer. For example, a filling port can be connected to and in fluid communication with one or more inflatable chambers of the spacer via a filling tube. As such, in use, a fluid transfer mechanism (*e.g.,* a pump and/or valve) can be connected to the filling port, and upon actuation, introduce the one or more filling materials into the one or more inflatable chambers of the spacer by way of the filling tube. As used herein, "fluid transfer" encompasses transfer of the one or more filling materials. Non-limiting examples of suitable filling materials include one or more gases, one or more liquids, one or more gels, or any combination thereof *(e.g.,* air, saline, hyaluronic acid, and/or silicone).

The terms "filled" or "inflated" are intended to mean that the spacer or inflatable chamber(s) thereof has/have filler material therein or added to it in a desired amount or pressure. These terms are not intended to mean that the spacer or inflatable chamber(s) is/are necessarily entirely or 100% filled with a filler material when the inflatable chamber(s) are "inflated" or when the spacer is in an "inflated" state (however, these are within the scope of the term "filled"). Similarly, the term "deflated" does not necessarily mean that the spacer or inflatable chamber(s) thereof is/are entirely empty or at 0 pressure. There may be some filler material and the chamber may have a non-zero pressure in a "deflated" inflatable chamber or when the spacer is in a "deflated" state. A "deflated" spacer or inflatable chamber(s) is/are intended to mean that the spacer or inflatable chamber(s) does/do not include the filler material in an amount or at a pressure that would be desired after the spacer or inflatable chamber(s) is/are filled.

The spacer can include two or more inflatable chambers. The two or more inflatable chambers can allow the spacer to have different portions with different stiffness and/or shapes. In this way, the spacer can be further tailored to a shape that enhances the positional stability of the spacer within different locations within a joint. For example, for subacromial use, the superior aspect of the spacer can be stiffer compared to the inferior portion of the spacer. Inflation of both superior and inferior chambers can enhance the proximal-distal (superior-inferior) stability of the humeral head within the glenoid concavity, whereas higher stiffness in the superior chamber can bias the position of the humerus to remain more inferiorly placed which can aid the elevation of the arm through activation of the deltoids, among other muscles. In addition, the humeral head has a much bigger range of motion than the scapula. A softer inferior portion of the spacer can allow for larger relative motion of the humeral head without dragging the spacer excessively, whereas a stiffer superior portion of the spacer can provide a higher interlock to the acromion, resulting in better positional stability of the spacer relative to the body.

The spacer can be formed of a variety of biocompatible materials. A person skilled in the art will appreciate that the type of material(s) can depend at least upon the targeted use of the spacer. For example, the spacer can be formed of one or more absorbable materials, or the spacer can be formed of one or more non-absorbable materials. The spacer can be formed of a combination of one or more absorbable materials and one or more non-absorbable materials.

The spacer can have a variety of configurations that allow the spacer to mechanically interlock with a portion of the anatomy (*e.g.,* the acromion) to thereby self-anchor to and inhibit migration from the joint. For example, the spacer can be substantially dog-bone shaped, substantially U-shaped, or substantially dome-shaped. A person skilled in the art will appreciate that the particular structural configuration of the spacer depends at least upon the implant site (*e.g.,* within a joint) and the anatomy of the patient. For example, for subacromial use, the spacer can be shaped such that it fits around the acromion rather than simply being held against the rotator cuff by the acromion. As such, the spacer can be shaped so as to center the humeral head and resist superior, anterior, and/or posterior positioning of the humerus relative to the scapula. Alternatively, or in addition, at least one surface of the spacer can be textured to further inhibit migration of the spacer when the spacer is implanted.

Where the spacer is substantially dome-shaped, the spacer, in an inflated state, can have a shape that is less than a hemispherical portion of a hollow sphere and includes a convex side and a concave side. The concave side of the spacer can be positioned opposite a portion of the acromion such that the concavity aids in holding the spacer in place, and the convex side of the spacer would articulate with the soft tissue at the superior aspect of the humerus and urge it against superior migration.

The spacer can be configured to be drug-eluting (*e.g.,* immediate release or extended release). For example, the spacer can be formed of a semi-permeable material and at least one medicament can be added to the filling material such that, when inflated, the spacer can be configured to release the at least one medicament (*e.g.,* while the spacer is implanted). Where the spacer is formed of an absorbable material, the at least one medicament can be released from the spacer (*e.g.,* from one or more inflatable chambers of the spacer) when the spacer begins to break down while the spacer is implanted. The at least one medicament can be incorporated within the one or more materials forming the spacer itself such that, for example, the at least one medicament can be released over time (*e.g.,* extended release). The at least one medicament can be any suitable medicament(s). Non-limiting examples of suitable medicaments include one or more medicaments that have anti-inflammatory, anti-clotting, analgesic, anti-infective, anti-biofilm, anti- or pro-bioadhesion, and/or growth factor properties.

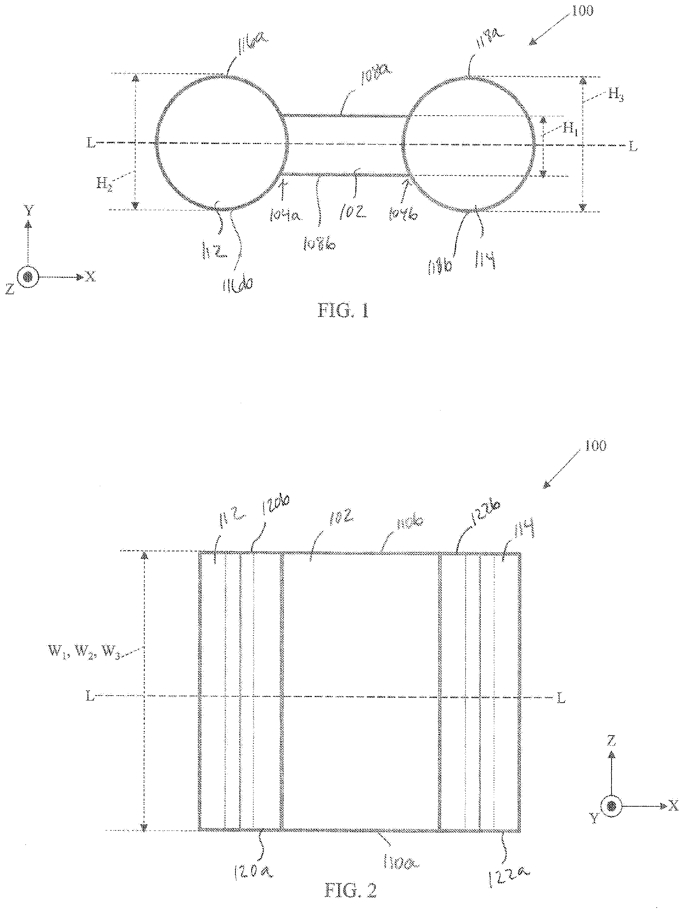

FIGS. 1 and 2 illustrate an implant that is a spacer 100. The spacer 100 is configured to be implanted within a joint (*e.g.,* a shoulder joint), and, when implanted, the spacer 100 is configured to engage with a portion of the anatomy to thereby self-anchor itself to the joint. This self-anchoring can inhibit migration of the spacer relative to the joint. While the spacer 100 is configurable between a deflated state and an inflated state, the spacer 100 is illustrated in an inflated state.

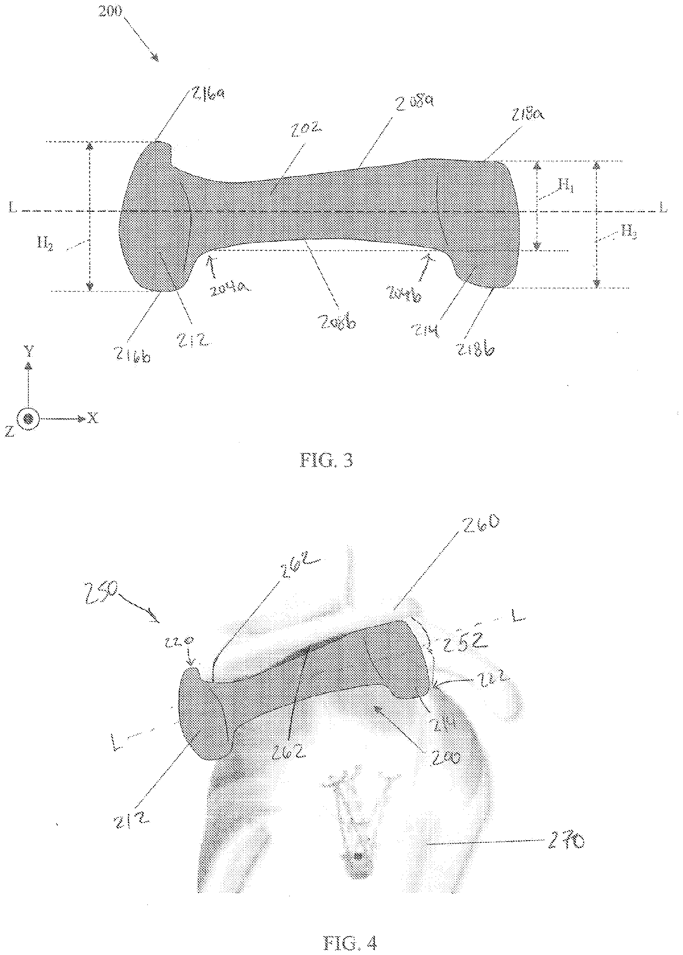

The spacer 100 includes an elongated central body 102 extending from a first end 104a to a second end 104b with a longitudinal axis L extending therebetween (*e.g.,* in the X-direction). The elongated central body 102 has a height H₁ in a direction *(e.g.,* the Y-direction) that is transverse to the longitudinal axis L. The maximum height of the elongated central body 102 is the greatest distance between the top-most surface 108a and the bottom-most surface 108b of the elongated central body 102. As shown, the height H₁ of the elongated central body 102 is generally consistent (*e.g.,* nominally identical within manufacturing tolerances) along its length (*e.g.,* extending along the longitudinal axis L; in the X-direction), and therefore the height H₁ of the elongated central body 102 is equal to the maximum height of the elongated central body 102. As shown in FIG. 3, the height of the elongated central body can vary along its length.

As further shown in FIG. 2, the elongated central body 102 has a width W₁ that extends in a direction *(e.g.,* the Z-direction) that is transverse to the longitudinal axis L. The maximum width of the elongated central body 102 is the greatest distance between the front and back surfaces 110a, 110b of the elongated central body 102. In this illustrated implant, the width W₁ of the elongated central body 102 is generally consistent (*e.g.,* nominally identical within manufacturing tolerances) along its length, and therefore the width W₁ of the elongated central body 102 is equal to the maximum width of the elongated central body 102. In other implants, the width of the elongated central body 102 can vary along its length. A person skilled in the art will appreciate the dimensions (*e.g.,* height, width, and length) of the elongated central body depend at least upon the intended implant site and the anatomy of the patient at such site *(e.g.,* the anatomy of the joint in which the spacer is to self-anchor to).

The spacer 100 also includes first and second wings 112, 114 extending from the first and second ends, 104a, 104b, respectively, of the elongated central body 102. While the first and second wings 112, 114 can have a variety of configurations, in this implant, the first and second wings 112, 114 have a substantially cylindrical shape. Further, the first and second wings 112, 114 are generally uniform (*e.g.,* nominally identical within manufacturing tolerances) in shape and size. As shown, the height of the first varies along its length (*e.g.,* extending along the longitudinal axis L; in the X-direction), and therefore, the maximum height H₂ of the first wing 112 is the greatest distance between top and bottom surfaces 116a, 116b of the first wing 112. Similarly, the height H₂ of the second wing 114 varies along its length (*e.g.,* extending along the longitudinal axis L; in the X-direction), and therefore, the maximum height H₃ of the second wing 114 is the greatest distance between top and bottom surfaces 118a, 118b of the second wing 114. While the maximum heights H₂, H₃ of the first and second wings 112, 114 can vary relative to each other, in this illustrated implant, the maximum height H₂ of the first wing 112 and the maximum height H₃ of the second wing 114 are substantially equal (*e.g.,* nominally identical within manufacturing tolerances). Further, in this illustrated implant, the first and second wings 112, 114 have a maximum height H₂, H₃ that is greater than the maximum height H₁ of the elongated central body 102. As a result, the spacer 100 is substantially dog-boned shape.

As further shown in FIG. 2, the first wing 112 has a width W₂ that extends in a direction (*e.g.,* the Z-direction) that is transverse to the longitudinal axis L. The maximum width of the first wing 112 is the greatest distance between the front-most surface 120a and the back-most surface 120b of the first wing 112. In this illustrated implant, the width W₂ of the first wing 112 is generally consistent (*e.g.,* nominally identical within manufacturing tolerances) along its length, and therefore the width W₂ of the first wing 112 is equal to the maximum width of the first wing 112. Similarly, the second wing 114 has a width W₃ that extends in a direction (*e.g.,* the Z-direction) that is transverse to the longitudinal axis L. The maximum width of the second wing 114 is the greatest distance between the front-most surface 122a and the back-most surface 122b of the second wing 114. In this illustrated implant, the width W₃ of the second wing 114 is generally consistent (*e.g.,* nominally identical within manufacturing tolerances) along its length, and therefore the width W₃ of the second wing 114 is equal to the maximum width of the second wing 114. A person skilled in the art will appreciate the dimensions (*e.g.,* height, width, and length) of the first wing and the second wing depend at least upon the intended implant site and the anatomy of the patient at such site (*e.g.,* the anatomy of the joint within which the spacer is to self-anchor).

FIG. 3 illustrates an implant that is a spacer 200. The spacer 200 that is configured to be implanted within a joint *(e.g.,* a shoulder joint), and, when implanted, the spacer 200 is configured to engage with a portion of the anatomy to thereby self-anchor itself to the joint. While the spacer 200 is configurable between a deflated state and an inflated state, the spacer 200 is illustrated in an inflated state. Aside from the differences described in detail below, the spacer 200 can be similar to spacer 100 (FIGS. 1 and 2) and therefore common features are not described in detail herein.

The spacer 200 includes an elongated central body 202 extending from a first end 204a to a second end 204b with a longitudinal axis L extending therebetween. The elongated central body 202 has a height that extends in a direction (*e.g.,* the Y-direction) that is transverse to the longitudinal axis L. As shown, the height of the elongated central body 202 varies along its length (*e.g.,* extending along the longitudinal axis L; in the X-direction). As such, the first maximum height H₁ of the elongated central body 202 is the greatest distance between top-most surface 208a and the bottom-most surface 208b of the elongated central body 202. A person skilled in the art will appreciate the dimensions (*e.g.,* height, width, and length) of the elongated central body depend at least upon the intended implant site and the anatomy of the patient.

The spacer 200 also includes first and second wings 212, 214 extending from the first and second ends 204a, 204b of the elongated central body 202, respectively. While the first and second wings 212, 214 can have a variety of configurations, each of the first and second wings 212, 214 have a maximum height H₂, H₃ that is greater than the maximum height H₁ of the elongated central body 202. As a result, the spacer 200 is substantially dog-boned shape.

As shown, the height of the first wing 212 varies along its length (*e.g.,* extending along the longitudinal axis L; in the X-direction). As such, the maximum height H₂ of the first wing 212 is the greatest distance between the top-most surface 216a and the bottom-most surface 216b of the first wing 212. Similarly, the height of the second wing 214 varies along its length *(e.g.,* extending along the longitudinal axis L; in the X-direction). As such, the maximum height H₃ of the second wing 214 is the greatest distance between the top-most surface 218a and the bottom-most surface 218b of the second wing 214. While the maximum heights H₂, H₃ between the first and second wings 212, 214 can vary relative to each other, in this illustrated implant, the maximum height H₂ of the first wing 212 is greater than the maximum height H₃ of the second wing 214. In other implants, the maximum height of the first wing 212 can be less than the maximum height of the second wing 214. In yet other implants, the height of the first wing 212 and/or the second wing 214 can be generally consistent (*e.g.,* nominally identical within manufacturing tolerances) along its respective length. A person skilled in the art will appreciate the dimensions (*e.g.,* height, width, and length) of the first and second wings depend at least upon the intended implant site and the anatomy of the patient.

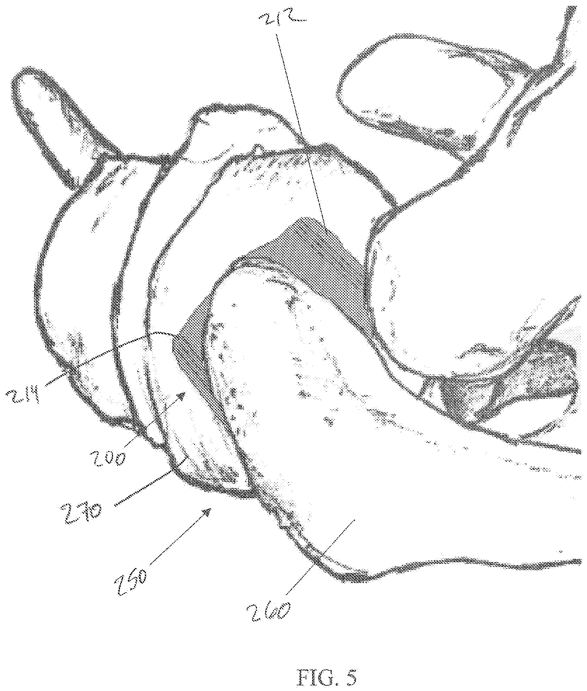

FIGS. 4 and 5 illustrate the spacer 200 implanted within the subacromial space 252 of a shoulder joint 250 and thus between an acromion 260 and a humerus 270. As shown, when the spacer 200 is implanted in a deflated state and subsequently inflated to an inflated state, the elongated central body 202 extends underneath the acromion 260 and superior to the humerus 270. Further, a portion 220 *(e.g.,* a top portion) of the first wing 212 is curved upward and away from the longitudinal axis L of the elongated central body 202 such that the portion 220 of the first wing 212 extends above the deep-most surface 262 of the acromion 260. As such, the spacer 200 partially encircles the acromion 260. As a result, the first wing 212 can mechanically interlock with a portion of the anatomy, which in this example, is the acromion 260. That is, the first wing 212 is shaped such that the engagement between the spacer 200 itself and the bone configuration of the acromion 260 allows the spacer 200 to self-anchor to the shoulder joint 250. Thus, the position of the spacer 200 can be maintained and the spacer 200 can be inhibited from migrating.

In certain implants, for example, as shown in FIG. 4, a portion 222 *(e.g.,* a bottom portion) of the second wing 214 can be curved downward and away from the longitudinal axis L of the elongated central body 202. As such, the portion 222 of the second wing 214 can engage the humerus 270 in such a way to further help the spacer 200 to self-anchor to the shoulder joint 250.

Alternatively, or in addition, a portion *(e.g.,* a top portion) of the second wing can be curved upward and away from the longitudinal axis of the elongated central body such that the portion of the second wing extends above a deep-most surface of the acromion when the spacer is implanted and in the inflated state. This allows the second wing to mechanically interlock with a portion of the anatomy.

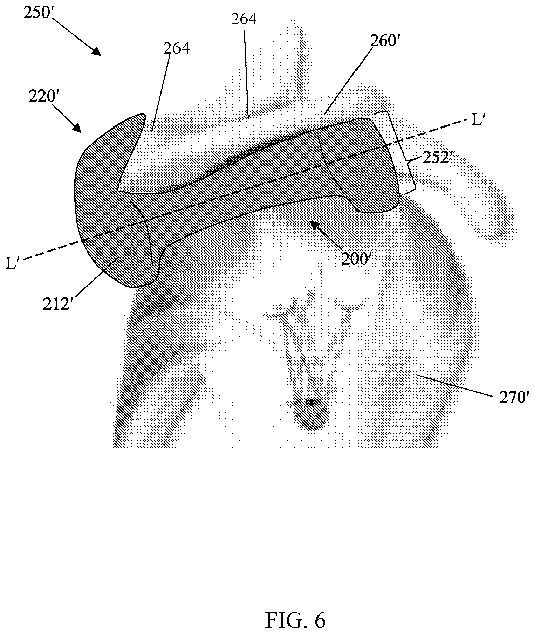

In other implants, a portion of at least one of the first wing and the second wing is curved upward and away from the longitudinal axis of the elongated central body such that the portion of the at least one of the first wing and the second wing is configured to extend flush to or above a superficial-most surface of the acromion when the spacer is implanted and in an inflated state. For example, as shown in FIG. 6, a spacer 200' is implanted within a subacromial space 252' of a shoulder joint 250' and thus between an acromion 260' and a humerus 270'. The spacer 200' is similar to spacer 200 of FIGS. 3-5, except that the portion 222' of the first wing 212' is further curved upward and away from the longitudinal axis L' such that the portion 220' of the first wing 212' extends above a superficial-most surface 264 of the acromion 260'.

The spacer can be U-shaped such that, when implanted within a joint, the elongated central body runs underneath the acromion, the first wing is positioned on one side of the acromion, and the second wing is positioned on the other side of the acromion. As such, the spacer can partially encircle the acromion. While the first and second wings can have a variety of configurations, the first and second wings can each have a height that is greater than the height of the elongated central body. As such, the elongated central body is thinner in height relative to the first and second wings. Further, the first and second wings can each have a different width and/or height, in which the width and/or height of the first wing is less than the width and/or height of the second wing.

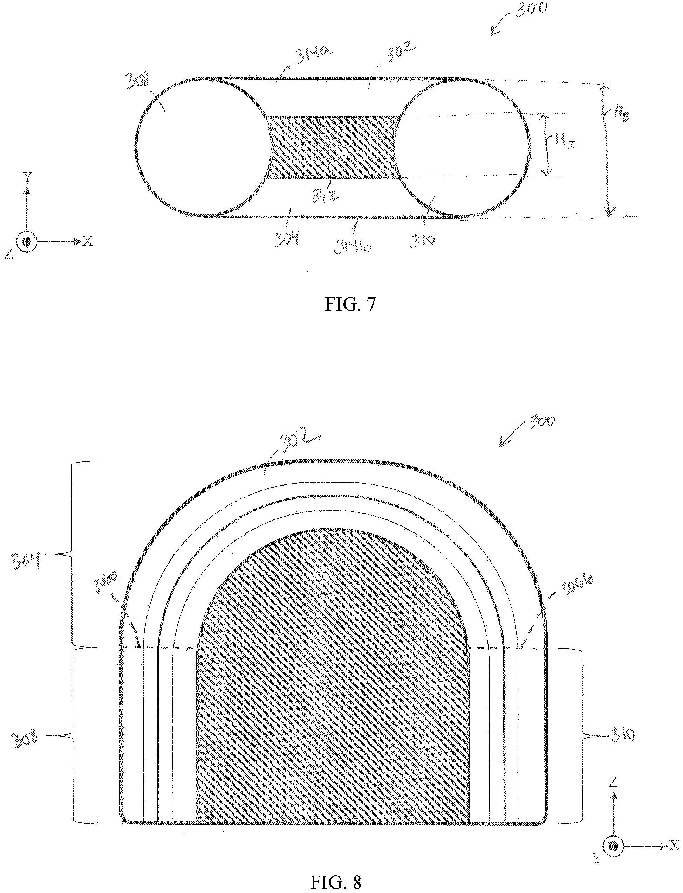

FIGS. 7 and 8 illustrate another implant that is a spacer 300. The spacer has an elongated outer body 302 that includes a central segment 304 extending from a first end 306a to a second end 306b, a first wing 308 that extends outward from the first end 306a, and a second wing 310 that extends outward from the second end 306b. In this illustrated implant, the elongated outer body 302 is U-shaped and defines an inner portion 312 of the spacer 300. As shown in FIG. 7, the height H_{I} (*e.g.,* extending in the Y-direction) of the inner portion 312 is less than the maximum height H_{B} of the of the elongated outer body 302. The maximum height H_{B} of the elongated outer body 302 extends from the top-most surface 314a to the bottom-most surface of 314b. Thus, the inner portion 312 is thinner such that, when the spacer 300 is implanted within a joint, *e.g.,* the subacromial space therein, at least a portion of the inner portion 312 can be positioned underneath the acromion. Further, when implanted, the first wing 308 can be positioned on one side of the acromion, and the second wing 310 can be positioned on the other side of the acromion. It is also contemplated herein that the inner portion can be partially or completely omitted.

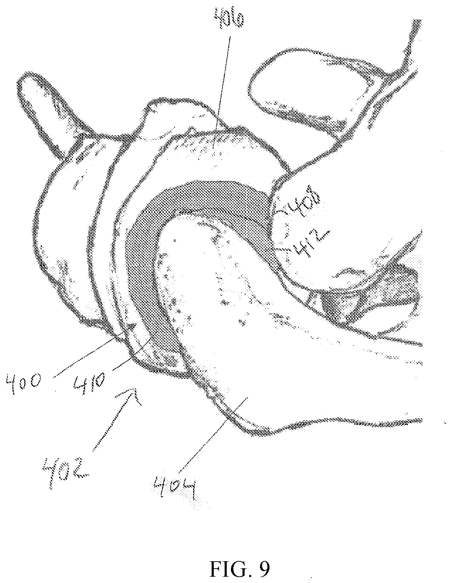

FIG. 9 illustrates another implant that is a spacer 400. The spacer 400 is implanted within a shoulder joint 402 between the acromion 404 and the humerus 406. The spacer 400 is substantially similar to spacer 300 in FIGS. 7 and 8 and therefore common features are not described in detail herein. As shown, the first wing 408 (partially obstructed) is positioned on one side of the acromion 404, the second wing 410 (partially obstructed) is positioned on the other side of the acromion 404, and the majority of the inner portion 412 is positioned underneath the acromion 404.

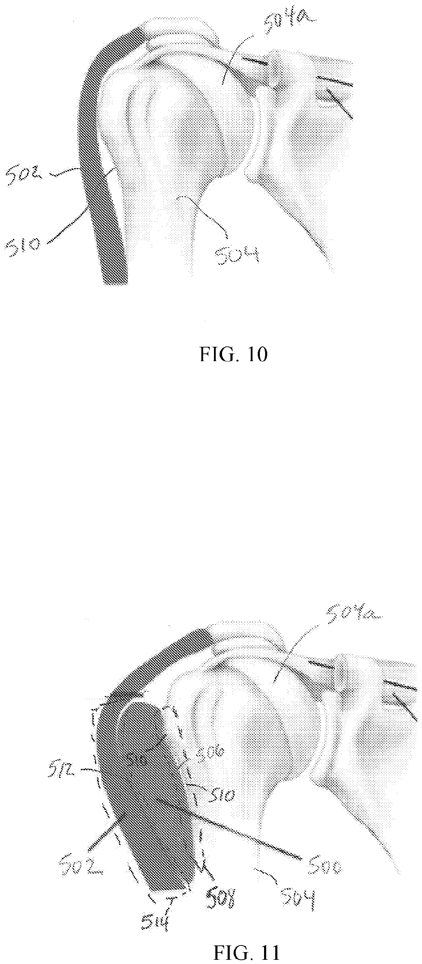

As mentioned above, the present spacers can be designed to alter the muscle biomechanics in instances where a muscle may be dysfunctional or have insufficient strength (see FIG. 10). In such instances, a spacer as disclosed herein can be used for muscle augmentation. For example, in some examples, a spacer in a deflated state (*e.g.,* a deflated spacer) can be positioned between the humerus and the deltoid, and then once positioned, the spacer can be inflated from the deflated state to an inflated state *(e.g.,* an inflated spacer (see FIG. 11). As a result, the implant causes the deltoid to stretch via lengthening, increase the muscle wrap around the humeral head to enhance the concavity compression on the glenoid fossa, and increase the distance between the muscle and the center of rotation of the arm (*e.g.,* increase the moment arm of the muscle). Further, the inflation of the spacer can allow the spacer to self-anchor between the humerus and the muscle without penetration into the humerus. The spacer can be positioned lateral to the humerus.

FIG. 10 illustrates a weak/dysfunction deltoid muscle 502 and FIG. 11 schematically illustrates deltoid function augmentation using a spacer 500 that is in an inflated state. As shown, the spacer 500 is positioned between the deltoid muscle 502 and the humerus 504. As a result, the spacer 500 stretches the deltoid muscle 502 and increases compression around the humeral head 504a of the humerus 504. Further, as shown in FIG. 11, the spacer 500 is structured in such a way that allows the spacer 500 to self-anchor between the deltoid muscle 502 and the humerus 504 without any penetration of the humerus 504. In this illustrated example, the spacer 500 has an elongated oblong shape with an outer-most first surface 506 that runs along and conforms to the shape of an upper portion 508 of a lateral surface 510 of the humerus 504, and an outer-most second surface 512 that has a convex-shape that runs along an upper portion 514 of the deltoid muscle 502. As shown, when implanted, the outer-most first surface 506 of the spacer 500 engages with the humerus 504 and the outer-most second surface 512 engages with the deltoid muscle 502 to thereby allow the spacer 500 to self-anchor therebetween. While the spacer 500 is illustrated with respect to the deltoid muscle, in other examples, the spacer can be used for augmenting other shoulder muscles, such as the subscapularis, the infraspinatus, the latissimus dorsi, and the pectoralis major.

The present spacers can be designed to be positioned between muscles (*e.g.,* intermuscular implantation) or muscle fibers (*e.g.,* intramuscular implantation) to augment the muscle(s) or muscle fibers so as to improve muscle function. For example, a spacer in a deflated state (*e.g.,* a deflated spacer) can be positioned between portions of a muscle, and then once positioned, the spacer can be inflated from the deflated state to an inflated state *(e.g.,* an inflated spacer). As a result, the implant locally redirect portions of the muscle fibers away from one another to affect the result of muscle contraction. Further, the inflation of the spacer can allow the spacer to self-anchor within the muscle.

Further, shoulder pain can be multifactorial and can happen separately at different times *(e.g.,* pain during sleep vs. pain related to daytime activities). Accordingly, multiple spacers can be placed at different respective strategic locations at or around the shoulder joint(s) and each spacer can be configured to stabilize the shoulder to address a specific activity (*e.g.,* sleeping and day time activities, lying on one's side vs. lying on one's back, and sitting activities vs. standing activities). For example, a subacromial spacer *(e.g.,* a spacer positioned within the subacromial space) can benefit the patient during daily or rehabilitation activities, while a posterior support spacer *(e.g.,* a spacer that is positioned against posterior side of the humeral head) at the humeral head can alleviate pain during sleep. As such, implant systems have been in development that can concurrently address multifactorial shoulder pain.

In general, an implant system can include at least two spacers, each having at least one inflatable chamber defined therein, a fluid passageway extending between the inflatable chambers, and a valve positioned within the fluid passageway. Since the inflatable chambers are in fluid communication with one another via the fluid passageway, their respective volumes can be selectively adjusted (*e.g.,* auto-adjusted, *e.g.,* via a controller and software, or manually adjusted) relative to each other to allow for different chamber configurations (*e.g.,* deflated or inflated positions). These different configurations can be based on the position of the shoulder joint. As such, a volume of a given inflatable chamber can be selected based on a specific activity and the appropriate volume is tailored to stabilize the shoulder joint and therefore alleviate pain during such activity.

Once implanted, calibration profiles can be created (*e.g.,* by the health care provider) for different positions of the patient's shoulder joint, and thus for specific activities. These calibration profiles can be used to regulate fluid transfer, and thus pressurization, between the inflatable chambers of the spacers. A person skilled in the art will appreciate that fluid transfer into and out of the respective inflatable chambers will result in a change in pressure within the respective inflatable chambers. That is, fluid transfer into an inflatable chamber, increases, and thus pressurizes, the inflatable chamber, whereas fluid transfer out of the inflatable chamber, decreases, and thus depressurizes, the inflatable chamber.

The inflatable chambers can include one or more pressure sensors configured to communicate wirelessly with a receiver located outside of the body. The calibration profiles can be maintained on the receiver and employed as set points in the context of a pressure feedback control system, including the one or more pressure sensors, the valve(s), and any pumps, if present. These set points can be associated with a respective specific shoulder position, and thus, respective specific activity, such as sleeping or daytime or rehabilitation activities. Alternatively, during a calibration session, the patient in consultation with the health care provider can determine pressures of the inflatable chambers that "feel" correct for stabilization and alleviation of pain during respective activities. As such, the patient can therefore rely on their memory, and thus, manually control fluid transfer between the individual inflatable chambers. The fluid transfer, and thus, pressure regulation, can be performed in an automated manner (*e.g.,* by using one or more pressure sensors and an automated pump and/or valve) or a manual manner (*e.g.,* patient movement of their shoulder or upper arm and/or by patient actuation of a valve or pump). Thus, the inflation scheme of the inflatable chambers for pain can be readily adjusted by pressurizing and depressurizing the inflatable chambers until pain relief is achieved.

In instances where the implant system is implanted into a patient with weak or dysfunctional shoulder muscles, the health care provider can calibrate the proper inflation pressures based on physical examination and the expected activities. Inflation profiles can be saved in a remote receiver, or a description of the profiles can be provided for the patient to reproduce. During use, fluid transfer of the inflatable chambers can be regulated to adjust the pressures of the inflatable chambers to the appropriate profile suited to an activity by activating the respective valves and pump, if present, of the implant system.

In instances wherein the implant system is implanted into a patient with multiple problems, such as anterior instability, posterior instability, and/or arm abduction deficit, the health care provider can help develop different pressure profiles to address the problems and save the profiles in a remote receiver. During use, when an uncomfortable position or activity is experienced by the patient, activation of the valves and pump, if present, of the implant system can be used to restore the appropriate pressure profile.

The implant system can be implanted subcutaneously. In such examples, the valve can be a mechanical valve that can be opened by applying a finger pressure across the skin. In other examples, the valve can be operated magnetically across the skin, similar to the manner of a hydrocephalus valve. Additional details on hydrocephalus valves can be found in U.S. Patent No. 8,591,499. In yet other examples, the valve can be a pressure limiting duckbill type valve, so that when an inflatable chamber is inflated, and thus pressurized, the valve opens at a predetermined pressure. This type of valve can also prevent inadvertent over-pressuring of the inflatable chamber by acting as a relief valve. With the valve open, the patient can move their arm to induce fluid transfer between the inflatable chambers of the spacers, and then close the valve once a desired configuration is obtained.

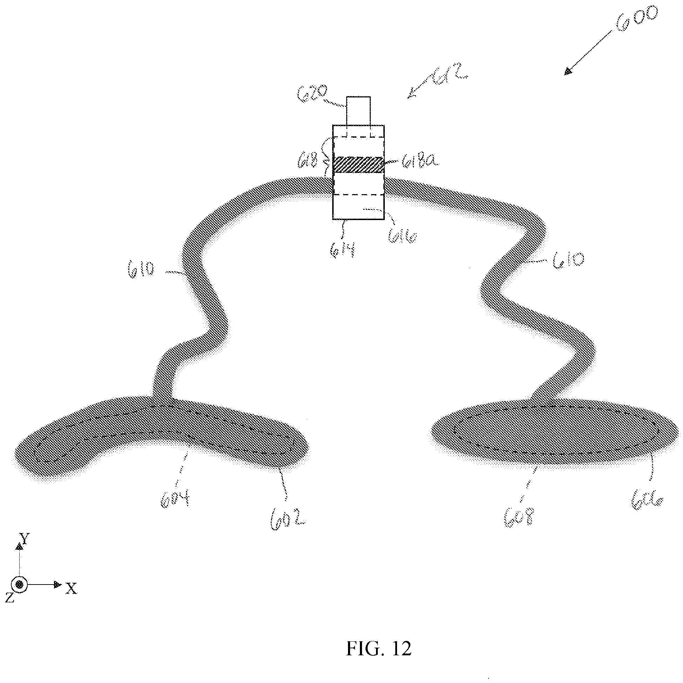

FIG. 12 illustrates an implant system 600 having a first spacer 602 with a first inflatable chamber 604 defined therein, a second spacer 606 with a second inflatable chamber 608 defined therein, a fluid passageway 610 extending between the first and second inflatable chambers 604, 608, and a valve 612 positioned within the fluid passageway 610. The first and second inflatable chambers 604, 608 are each configured to reversibly move between respective volumes. For example, the first inflatable chamber 604 can be configured to reversibly move between a first volume and a second volume, and the second inflatable chamber 608 can be configured to reversibly move between a third volume and a fourth volume. The volume movement between the first and second inflatable chambers 604, 608 can occur when the valve 612 is an open position *(e.g.,* a second state). Further, as shown, the first and second inflatable chambers 604, 608 have different shapes relative to each other, whereas in other examples, the first and second inflatable chambers 604, 608 can have generally uniform shapes (*e.g.,* nominally identical within manufacturing tolerances). A person skilled in the art will appreciate that the dimensions and shape of the first and second inflatable chambers depend at least upon the implantation site and the anatomy of the patient.

The valve 612 includes a valve body 614 having a valve chamber 616 defined therein, a plug 618 disposed within the valve chamber 616 with a channel 618a extending therethrough, and an actuator 620 coupled to the plug 618. The valve 612 can be biased to a closed position *(e.g.,* a first state) via a biasing element (not shown). In use, when the valve 612 is in a closed position (see FIG. 12), the channel 618a of the plug 618 is misaligned with the fluid passageway 610, and therefore fluid transfer between the first and second inflatable chambers 604, 608 is inhibited. As such, movement between the respective volumes of the first and second inflatable chambers 604, 608 is substantially prevented.

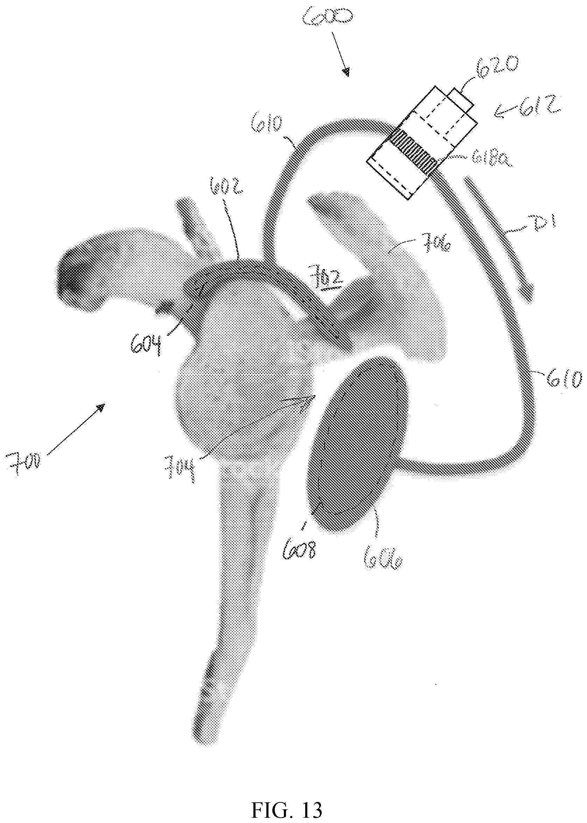

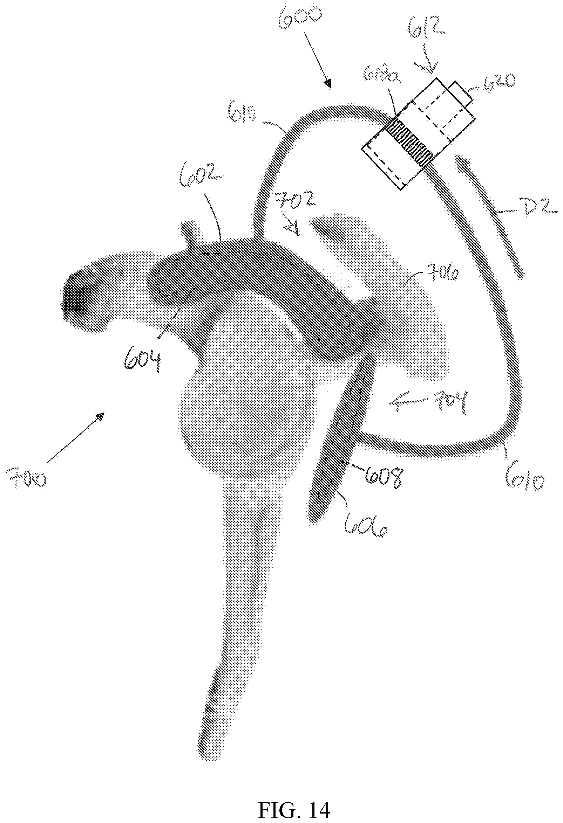

Further, the valve 612 can move from the closed position *(e.g.,* a first state) to an open position (*e.g.,* a second state) via actuation of the actuator 620 of the valve 612. When the actuator 620 is actuated (*e.g.,* manually, *e.g.,* by a finger or hand pressure provided by the patient, or automatically via a controller and/or software), the actuator 620 is configured to translate the plug 618 in a downward direction (*e.g.,* in a Y-direction) to cause the channel 618a of the plug 618 to come into at least partial alignment with the fluid passageway 610. This alignment allows one or more filling materials to pass through the valve 612, and therefore, to pass through the fluid passageway 610. As such, when the valve 612 is in the open position, the channel 618a forms part of the fluid passageway 610. Since the fluid passageway 610 extends between the first and second inflatable chambers 604, 608, fluid transfer can therefore occur between the first and second inflatable chambers 604, 608 (see FIGS. 13 and 14) when the valve 612 is in the open position. This fluid transfer can be based on a position of a joint *(e.g.,* a shoulder joint), and thus, can be based on a specific activity (*e.g.,* sleeping or daytime activities).

One or more filling materials can be present in at least one of the inflatable chambers prior to implantation. For example, the one or more filling materials is present within the first inflatable chamber 604 and the second inflatable chamber 608. As described in more detail below, in use, this fluid transfers between the first and second inflatable chambers 604, 608 such that the configurations of the spacers 602, 606 can change to thereby effect desirable support to the joint when the patient experiences an uncomfortable position or activity.

FIGS. 13 and 14 illustrate the first spacer 602 being positioned within a first space 702 of a joint 700 and a second spacer 606 being positioned within a second space 704 of the joint 700. In this illustrated example, the joint 700 is a shoulder joint. For simplicity purposes, only the scapula of the shoulder joint is being illustrated in FIGS. 13 and 14. A person skilled in the art will appreciate that the present implant systems can be configured to be used with other joints *(e.g.,* in the patella-femoral and/or tibio-femoral compartments in the knee, in the femoro-acetabular articulation at the hip, in the talar-maleolar articulation in the ankle, and/or other joints), and therefore, the present implant systems are not limited to the joints or spaces within the joints that are illustrated in the figures.

In this illustrated example, the first spacer 602 is configured to be positioned superior to the humerus head (not shown), and thus, between the acromion 706 and the humerus head. That is, the first space 702 is the subacromial space. The second spacer 606 is configured to be positioned posterior to the humeral head (not shown). In use, the valve 612 can be moved from a closed position (see FIG. 12) to an open position (see FIGS. 13 and 14). While not shown, the implant system 600 can be implanted underneath the skin, and therefore, the actuator 620 of the valve 612 can be actuated, for example, by a patient applying a finger pressure across the skin to translate the actuator 620 such that the valve 612 moves from the closed position to an open position. Further, when the valve 612 is open, fluid transfer and the direction of fluid flow can be induced by manipulation of the patients arm or shoulder. Other suitable mechanisms (*e.g.,* manual or automated) for moving the valve between closed and open positions are described above.

Depending on the position of the shoulder joint, and thus the specific activity, when the valve 612 is in an open position, the fluid transfer can occur between the first inflatable chamber 604 and the second inflatable chamber 608. For example, for a sleeping activity, as shown in FIG. 13, when the valve 612 is moved from the closed position to the open position, the direction of fluid flow D1 is from the first inflatable chamber 604 to the second inflatable chamber 608, such that the second inflatable chamber 608 is inflated *(e.g.,* the second inflatable chamber 608 moves from a first volume to a second volume that is greater than the first volume) to provide a desirable amount of posterior support to the humerus (*e.g.,* by pushing the humeral head anteriorly or ventrally when a patient is lying on their back) to minimize patient pain during sleep. Further, this direction of fluid flow D1 causes the first inflatable chamber 604 to deflate *(e.g.,* the first inflatable chamber 604 moves from a first volume to a second volume that is less than the first volume). This configuration, however, may not be suitable for other activities.

For example, for a daily or rehabilitation activity, as shown in FIG. 14, when the valve 612 is moved from the closed position to the open position, the direction of fluid flow D2 *(e.g.,* in a direction opposite to D1) is from the second inflatable chamber 608 to the first inflatable chamber 604, such that first inflatable chamber 604 is inflated *(e.g.,* the first inflatable chamber 604 moves from a first volume to a second volume that is greater than the first volume) to provide a desirable amount of support in maintaining the subacromial space (*e.g.,* by compressing the humeral head downward) to minimize patient pain during a daily or rehabilitation activity (*e.g.,* by compressing the humeral head downward. Further, this direction of fluid flow D2 causes the second inflatable chamber 608 to deflate (*e.g.,* the second inflatable chamber 608 moves from a first volume to a second volume that is less than the first volume). Thus, the fluid transfer between the first and second inflatable chambers 604, 608 can be used to generate spacer configurations, and thus pressure profiles that are suited for different shoulder positions and activities so as to minimize or alleviate patient pain.

The implant system can also include at least one pump (*e.g.,* manually actuated or automated) that is in fluid communication with the fluid passageway. The at least one pump can be configured to drive fluid flow *(e.g.,* induce fluid transfer) between the first and second inflatable chambers when the valve is in an open position (*e.g.,* second state). For example, the fluid transfer can be performed using implantable pumps operated by hand pressure across the skin such as primer bulb pumps. In such examples, a multi-position valve can be used to control the direction of the flow. Where the implant system is needed for a relatively short period of time, the inflatable chambers may be accessed percutaneously via tubes and the fluid exchange mechanism (*e.g.,* pump(s) and valve (s)) is kept outside the body and directly accessible by the patient. This configuration can allow for greater flexibility in the implant system designs. For example, the fluid transfer may be directly managed via a displacement or syringe pump and a valve may not be needed. Alternatively, or in addition, the at least one pump can be configured to be operably coupled to the valve and configured to pump the one or more filling materials into the implant system prior to, concurrently, or subsequent to implantation of at least the spacers.

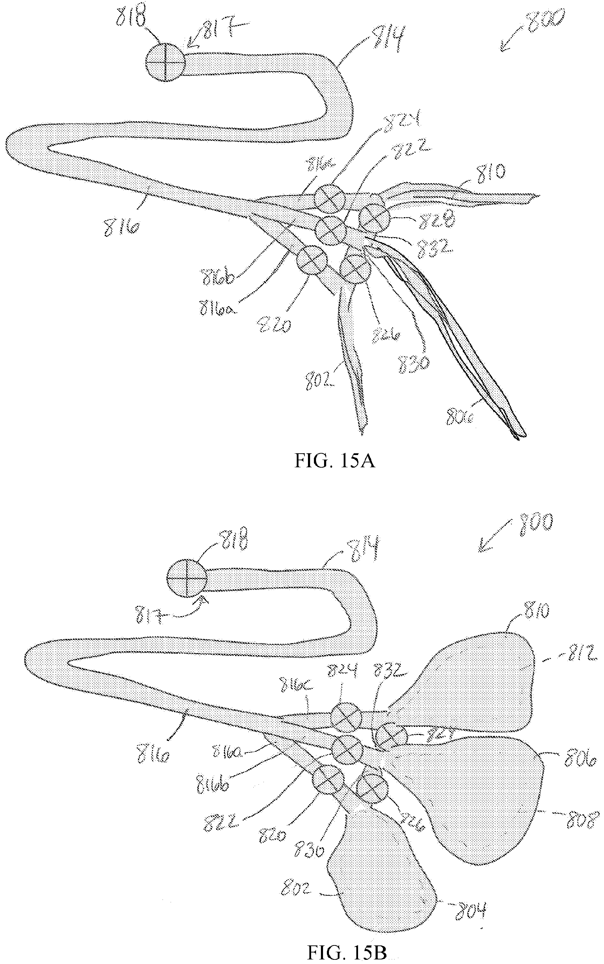

FIGS. 15A and 15B illustrate another implant system 800 having three spacers 802, 806, 810, a main fluid passageway 814, a main valve 818, three chambers valves 820, 822, 824, and two inter-chamber valves 826, 828. FIG. 15A illustrates each of the three spacers in a deflated state (*e.g.,* for implantation and/or removal from a joint, *e.g.,* a shoulder joint). FIG. 15B illustrates each of the three spacers 802, 806, 810 in an inflated state (*e.g.,* after implantation within a joint).

As shown in more detail in FIG. 15B, the first spacer 802 has a first inflatable chamber 804, the second spacer 806 has a second inflatable chamber 808, and the third spacer 810 has a third inflatable chamber 812. The first inflatable chamber 804 and the second inflatable chamber 808 are in fluid communication with each other via a first fluid passageway 830 extending therebetween. The first inter-chamber valve 826 is positioned within the first fluid passageway 830 and is configured to move between a closed position (*e.g.,* a first state) to an open position (*e.g.,* a second state). While the first inter-chamber valve 826 is generically illustrated, any suitable valve that be configured to control fluid transfer between the first and second inflatable chambers 804, 808 can be used.

Further, the second inflatable chamber 808 and the third inflatable chamber 812 are in fluid communication with each other via a second fluid passageway 832 extending therebetween. The second inter-chamber valve 828 is positioned within the second fluid passageway 832 and is configured to move between a closed position (*e.g.,* a third state) to an open position (*e.g.,* a fourth state). While the second inter-chamber valve 828 is generically illustrated, any suitable valve that be configured to control fluid transfer between the second and third inflatable chambers 808, 812 can be used.

The main fluid passageway 814 includes a central lumen 816 and three branched lumens 816a, 816b, 816c. The first branched lumen 816a extends between the central lumen 816 and the first inflatable chamber 804, the second branched lumen 816b extends between the central lumen 816 and the second inflatable chamber 808, and the third branched lumen 816c extends between the central lumen 816 and the third inflatable chamber 812. Further, one chamber valve of the three chamber valves 820, 822, 824 is positioned within a respective one branched lumen. While the three chamber valves 820, 822, 824 are each generically illustrated, any suitable valves that can be configured to control fluid transfer between the central lumen 816 and the respective inflatable chambers 804, 808, 812 can be used.

In this illustrated implant system, the first chamber valve 820 is positioned within the first branched lumen 816a, the second chamber valve 822 is positioned within the second branched lumen 816b, and the third chamber valve 824 is positioned within the third branched lumen 816c. As such, each chamber valve 820, 822, 824c is configured to control fluid transfer from the central lumen 816 to the respective inflatable chamber 804, 808, 812 (*e.g.,* move between a closed position (*e.g.,* a fifth state) and an open position (*e.g.,* a sixth state)). This allows the inflatable chambers 804, 808, 812 to have different inflation and deflation schemes for different shoulder positions, and thus, specific activities. For example, the inflatable chambers 804, 808, 812 can each be individually and separately inflated or delated, the inflatable chambers 804, 808, 812 can be concurrently inflated or deflated, two of the inflatable chambers can be concurrently inflated or deflated and the remaining inflatable chamber can be independently and separately inflated or deflated, or any combination thereof. As such, depending on the shoulder position, and thus the specific activity, the first inflatable chamber 804, the second inflatable chamber 808, and/or the third inflatable chamber 812, can be moved from a closed position to an open position or vice versa to help generate the desired configuration for the first spacer 802, the second spacer 806, and/or the third spacer 810.

As further shown in FIGS. 15A and 15B, the main valve 818 is positioned at the first end 817 (*e.g.,* the end farthest away from the inflatable chambers) of the central lumen 816 and is configured to move between a closed position (*e.g.,* a seventh state) and an open position (*e.g.,* an eighth position). While the main valve 818 is generically illustrated, any suitable valve that can be configured to control fluid transfer into and out of the central lumen 816 can be used.

In use, once the implant system 800 is implanted and the main valve 818 is an open position and operably connected to a fluid transfer mechanism (*e.g.,* a pump), one or more filling materials can be injected into the central lumen 816. Depending on the position of the chamber valves 820, 822, 824 (*e.g.,* open position), the first inflatable chamber 804, the second inflatable chamber 808, and/or the third inflatable chamber 812 can be inflated. Further, once the first inflatable chamber 804, the second inflatable chamber 808, and/or the third inflatable chamber 812 is inflated via injection of the one or more filling materials, depending on the position of the inter-chamber valves 826, 828 (*e.g.,* open position), fluid transfer between the first inflatable chamber 804, the second inflatable chamber 808, and/or the third inflatable chamber 812 can occur. Similarly, the main valve 818 in combination with the fluid transfer mechanism can be used to remove at least a portion of the one or more filling materials to thereby deflate the first inflatable chamber 804, the second inflatable chamber 808, and/or the third inflatable chamber 812 (*e.g.,* for the purposes of removing the implant system 800 from the patient).

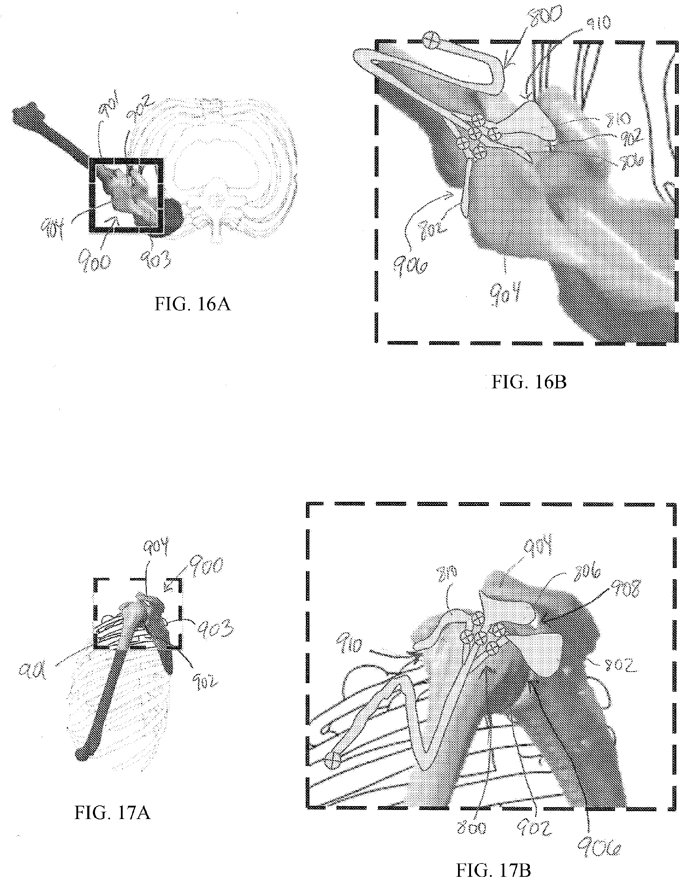

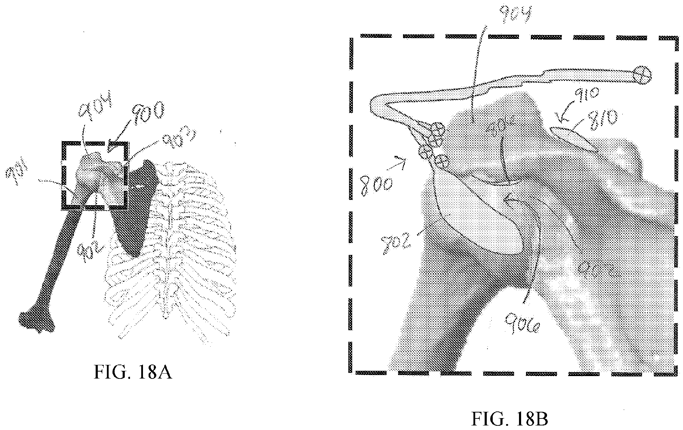

FIGS. 16A, 17A, and 18A each illustrate a shoulder joint anatomical environment having a left humerus 901 and scapula 903 of a joint 900. FIGS. 16B, 17B, and 18B illustrate the first spacer 802 being positioned within a first space 906 of a joint 900, the second spacer 806 being positioned with a second space 908 of the joint 900, and the third spacer 810 being positioned within a third space 910 of the joint 900. The first spacer 802 is configured to be positioned posterior to the humerus head 902, the second spacer 806 is configured to be positioned superior to the humerus head 902 (*e.g.,* between the humerus head 902 and the acromion 904), and the third spacer 810 is configured to be positioned anterior to the humerus head 902. As a result, the first inflatable chamber 804, the second inflatable chamber 808, and the third inflatable chamber 812 in combination can resist or eliminate undesired posterior, superior, and/or anterior positioning of the humerus 901 relative to the scapula 903. A person skilled in the art will appreciate that the present implant systems can be configured to be used with other joints (*e.g.,* in the patella-femoral and/or tibio-femoral compartments in the knee, in the femoro-acetabular articulation at the hip, in the talar-maleolar articulation in the ankle, and/or other joints), and therefore, the present implant systems are not limited to the joints or the spaces within the joints that are illustrated in the figures.

In the present disclosure, like-named components generally have similar features, and thus within a particular example each feature of each like-named component is not necessarily fully elaborated upon. Additionally, to the extent that linear or circular dimensions are used in the description of the disclosed implants, implant systems, and methods, such dimensions are not intended to limit the types of shapes that can be used in conjunction with such implants, implant systems, and methods. A person skilled in the art will recognize that an equivalent to such linear and circular dimensions can easily be determined for any geometric shape. Sizes and shapes of the implants and implant systems, and the components thereof, can depend at least on the anatomy of the subject in which the implants and implant systems will be used, the size and shape of components with which the implants and implant systems will be used, and the methods and procedures in which the implants and implant systems will be used.

Values or ranges may be expressed herein as "about" and/or from/of "about" one particular value to another particular value. When such values or ranges are expressed, other examples disclosed include the specific value recited and/or from/of the one particular value to another particular value. Similarly, when values are expressed as approximations, by the use of antecedent "about," it will be understood that here are a number of values disclosed therein, and that the particular value forms another example. It will be further understood that there are a number of values disclosed therein, and that each value is also herein disclosed as "about" that particular value in addition to the value itself. The term "about" can be used to mean, for example, within 10% of the recited value, within 5% of the recited value or within 2% of the recited value.

For purposes of describing and defining the present teachings, it is noted that unless indicated otherwise, the term "substantially" is utilized herein to represent the inherent degree of uncertainty that may be attributed to any quantitative comparison, value, measurement, or other representation. The term "substantially" is also utilized herein to represent the degree by which a quantitative representation may vary from a stated reference without resulting in a change in the basic function of the subject matter at issue.

One skilled in the art will appreciate further features and advantages of the invention. Accordingly, the invention is not to be limited by what has been particularly shown and described, except as indicated by the appended claims.

### The following relate to numbered aspects of the invention:

1. An implant, comprising:
   a spacer configured to be implanted within a joint, the spacer being configurable between a deflated state and an inflated state, the spacer having
      an elongated central body extending from a first end to a second end with a longitudinal axis extending therebetween, the elongated central body having a first maximum height in a direction transverse to the longitudinal axis, and
      first and second wings extending from the first and second ends of the elongated central body, respectively, each of the first and second wings having a maximum height greater than the first maximum height;
   wherein, when implanted and in the inflated state, at least one of the first wing and the second wing is configured to mechanically interlock with a portion of the anatomy to thereby self-anchor the spacer to the joint and inhibit migration of the spacer.
2. The implant of aspect 1, wherein the joint is a shoulder joint and the spacer is configured to be implanted between an acromion and a humerus of the shoulder joint and, when in the inflated state, the spacer is configured to at least partially encircle the acromion.
3. The implant of aspect 2, wherein a portion of at least one of the first wing and the second wing is curved upward and away from the longitudinal axis of the elongated central body such that the portion of the at least one of the first wing and the second wing is configured to extend above a deep-most surface of the acromion when the spacer is implanted and in the inflated state.
4. The implant of aspect 2, wherein a portion of at least one of the first wing and the second wing is curved upward and away from the longitudinal axis of the elongated central body such that the portion of the at least one of the first wing and the second wing is configured to extend flush to or above a superficial-most surface of the acromion when the spacer is implanted and in an inflated state.
5. The implant of aspect 1, wherein the spacer is substantially U-shaped.
6. The implant of aspect 1, wherein the spacer is substantially dog-bone shaped.
7. The implant of aspect 1, wherein at least one surface of the spacer is textured to thereby further inhibit migration of the spacer when the spacer is implanted.
8. The implant of aspect 1, wherein the spacer comprises at least one medicament disposed therein, and wherein the spacer is configured to release the at least one medicament while the spacer is implanted.
9. The implant of aspect 1, wherein the spacer is configured to receive at least one medicament when being inflated, and wherein the spacer is configured to release the at least one medicament while the spacer is implanted.
10. An implant system, the system comprising:
   a first spacer configured to be positioned within a first space of a joint, the first spacer having a first inflatable chamber defined therein that is configured to reversibly move between a first volume and a second volume;
   a second spacer being configured to be positioned within a second space of the joint, the second spacer having a second inflatable chamber defined therein that is configured to reversibly move between a third volume and a fourth volume;
   a first fluid passageway extending between the first and second inflatable chambers; and
   a first valve being positioned within the first fluid passageway, the first valve being configured to move between at least a first state and a second state that is different than the first state, wherein the first state inhibits fluid transfer between the first and second inflatable chambers to prevent movement between respective volumes, and wherein the second state allows a first fluid transfer to occur between the first and second inflatable chambers based on a first position of the joint such that the first and second inflatable chambers can reversibly move between respective volumes.
11. The system of aspect 10, further comprising at least one pump that is in fluid communication with the fluid passageway, wherein the at least one pump is configured to drive fluid flow between the first and second inflatable chambers when the at least one valve is in the second state.
12. The system of aspect 10, wherein the first valve is configured to facilitate a second, opposite fluid transfer between the first and second inflatable chambers based on a second position of the shoulder joint such that the first and second inflatable chambers can reversibly move between respective volumes, and wherein the second position is different than the first position.
13. The system of aspect 10, wherein the joint is a shoulder joint and the first space is posterior to a humeral head of the shoulder joint and the second space is superior or anterior to the humeral head.
14. The system of aspect 10, further comprising,
   a third spacer being configured to be positioned within a third space of the joint, the third spacer having a third inflatable chamber defined therein that is configured to reversibly move between a fifth volume and a sixth volume;
   a second fluid passageway extending between the second and third inflatable chambers; and
   a second valve being positioned within the second fluid passageway, the second valve being configured to move between at least a third state and a fourth state that is different than the third state, wherein the third state inhibits fluid transfer between the second and third inflatable chambers to prevent movement between respective volumes, and wherein the fourth state allows fluid transfer to occur between the second and third inflatable chambers based on at least one of the first position or a third position of the joint such that the second and third inflatable chambers can reversibly move between respective volumes.
15. The system of aspect 14, wherein the joint is the shoulder joint and the first space is posterior to a humeral head of the shoulder joint, the second space is superior to the humeral head, and the third space is anterior to the humeral head.
16. The system of aspect 14, further comprising a main fluid passageway having a central lumen and three branched lumens, wherein the first branched lumen extends between the central lumen and the first inflatable chamber, the second branched lumen extends between the central lumen and the second inflatable chamber, and the third branched lumen extends between the central lumen and the third inflatable chamber.
17. The system of aspect 16, further comprising at least one third valve that is positioned within at least one of the first branched lumen, the second branched lumen, and the third branched lumen, and wherein the at least one third valve is configured to move between a fifth state that inhibits fluid transfer therethrough and a sixth state that allows fluid therethrough.
18. The system of aspect 17, further comprising a fourth valve that is positioned at an end of the central lumen and configured to move between a seventh state and an eighth state, wherein, when in the eighth state, the fourth valve is configured to allow fluid transfer therethrough to at least one of inflate and deflate at least one of the first, second, and third inflatable chambers when the at least one third valve is in a sixth state.
19. A method for biomechanically augmenting muscle function, the method comprising:
   implanting a spacer in a deflated state between a humerus and a muscle; and
   inflating the spacer from the deflated state to an inflated state so as to allow the spacer to self-anchor between the humerus and the muscle without penetration into the humerus and to lengthen the muscle and increase the compression around a humeral head of the humerus.
20. The method of aspect 19, wherein the spacer is positioned lateral to the humerus.

## Claims

1. An implant, comprising:
a spacer configured to be implanted within a joint, the spacer being configurable between a deflated state and an inflated state, the spacer having
an elongated central body extending from a first end to a second end with a longitudinal axis extending therebetween, the elongated central body having a first maximum height in a direction transverse to the longitudinal axis, and
first and second wings extending from the first and second ends of the elongated central body, respectively, each of the first and second wings having a maximum height greater than the first maximum height;
wherein, when implanted and in the inflated state, at least one of the first wing and the second wing is configured to mechanically interlock with a portion of the anatomy to thereby self-anchor the spacer to the joint and inhibit migration of the spacer.

2. The implant of claim 1, wherein the joint is a shoulder joint and the spacer is configured to be implanted between an acromion and a humerus of the shoulder joint and, when in the inflated state, the spacer is configured to at least partially encircle the acromion.

3. The implant of claim 2, wherein a portion of at least one of the first wing and the second wing is curved upward and away from the longitudinal axis of the elongated central body such that the portion of the at least one of the first wing and the second wing is configured to extend above a deep-most surface of the acromion when the spacer is implanted and in the inflated state, or wherein a portion of at least one of the first wing and the second wing is curved upward and away from the longitudinal axis of the elongated central body such that the portion of the at least one of the first wing and the second wing is configured to extend flush to or above a superficial-most surface of the acromion when the spacer is implanted and in an inflated state.

4. The implant of claim 1, wherein the spacer is substantially U-shaped, or wherein the spacer is substantially dog-bone shaped.

5. The implant of any preceding claim, wherein at least one surface of the spacer is textured to thereby further inhibit migration of the spacer when the spacer is implanted.

6. The implant of any preceding claim, wherein the spacer comprises at least one medicament disposed therein, and wherein the spacer is configured to release the at least one medicament while the spacer is implanted.

7. The implant of any preceding claim, wherein the spacer is configured to receive at least one medicament when being inflated, and wherein the spacer is configured to release the at least one medicament while the spacer is implanted.

8. An implant system, the system comprising:
a first spacer configured to be positioned within a first space of a joint, the first spacer having a first inflatable chamber defined therein that is configured to reversibly move between a first volume and a second volume;
a second spacer being configured to be positioned within a second space of the joint, the second spacer having a second inflatable chamber defined therein that is configured to reversibly move between a third volume and a fourth volume;
a first fluid passageway extending between the first and second inflatable chambers; and
a first valve being positioned within the first fluid passageway, the first valve being configured to move between at least a first state and a second state that is different than the first state, wherein the first state inhibits fluid transfer between the first and second inflatable chambers to prevent movement between respective volumes, and wherein the second state allows a first fluid transfer to occur between the first and second inflatable chambers based on a first position of the joint such that the first and second inflatable chambers can reversibly move between respective volumes.

9. The system of claim 8, further comprising at least one pump that is in fluid communication with the fluid passageway, wherein the at least one pump is configured to drive fluid flow between the first and second inflatable chambers when the at least one valve is in the second state.

10. The system of claims 8 or 9, wherein the first valve is configured to facilitate a second, opposite fluid transfer between the first and second inflatable chambers based on a second position of the shoulder joint such that the first and second inflatable chambers can reversibly move between respective volumes, and wherein the second position is different than the first position.

11. The system of any of claims 8 to 10, wherein the joint is a shoulder joint and the first space is posterior to a humeral head of the shoulder joint and the second space is superior or anterior to the humeral head.

12. The system of any of claims 8 to 11, further comprising,
a third spacer being configured to be positioned within a third space of the joint, the third spacer having a third inflatable chamber defined therein that is configured to reversibly move between a fifth volume and a sixth volume;
a second fluid passageway extending between the second and third inflatable chambers; and
a second valve being positioned within the second fluid passageway, the second valve being configured to move between at least a third state and a fourth state that is different than the third state, wherein the third state inhibits fluid transfer between the second and third inflatable chambers to prevent movement between respective volumes, and wherein the fourth state allows fluid transfer to occur between the second and third inflatable chambers based on at least one of the first position or a third position of the joint such that the second and third inflatable chambers can reversibly move between respective volumes.

13. The system of claim 12, wherein the joint is the shoulder joint and the first space is posterior to a humeral head of the shoulder joint, the second space is superior to the humeral head, and the third space is anterior to the humeral head.

14. The system of claim 12 or 13, further comprising a main fluid passageway having a central lumen and three branched lumens, wherein the first branched lumen extends between the central lumen and the first inflatable chamber, the second branched lumen extends between the central lumen and the second inflatable chamber, and the third branched lumen extends between the central lumen and the third inflatable chamber.

15. The system of claim 14, further comprising at least one third valve that is positioned within at least one of the first branched lumen, the second branched lumen, and the third branched lumen, and wherein the at least one third valve is configured to move between a fifth state that inhibits fluid transfer therethrough and a sixth state that allows fluid therethrough, preferably, further comprising a fourth valve that is positioned at an end of the central lumen and configured to move between a seventh state and an eighth state, wherein, when in the eighth state, the fourth valve is configured to allow fluid transfer therethrough to at least one of inflate and deflate at least one of the first, second, and third inflatable chambers when the at least one third valve is in a sixth state.

16. The implant of claims 1 to 7, or implant system of claims 8 to 15, for use in a method of biomechanically augmenting muscle function.
